# FASCICULE DE BREVET EUROPEEN

(11) **EP 3 927 389 B1**
(45) Date de publication et mention de la délivrance du brevet: **03.05.2023**
(21) Numéro de dépôt: 20710235.1
(22) Date de dépôt: 18.02.2020
(51) Int. Cl.: A61L 31/10, A61L 31/06, A61L 31/04, A61L 15/22

(54) **MEMBRANE**
MEMBRAN
MEMBRANE

(30) Priorité: 22.02.2019 FR 1901812
(43) Date de publication de la demande: 29.12.2021
(73) Titulaire: Les Laboratoires Osteal Medical, 95957 Roissy Aeroport CDG (FR)
(72) Inventeur: BLANQUAERT, Daniel, 75008 PARIS (FR); LEROUX, Amélie, 95120 ERMONT (FR); MIGONNEY, Véronique, 95600 EAUBONNE (FR)
(74) Mandataire: CAPRI
(86) Numéro de dépôt international: PCT/FR2020/050290
(87) Numéro de publication internationale: WO 2020/169913

(56) Documents cités:
- WO-A1-2015/075397

## Description

La présente invention concerne une membrane et son procédé de fabrication. Cette membrane comprend au moins une nappe de textile formée de fibres réalisées à partir d'au moins un polymère. Ce type de membrane peut notamment être utilisé en vue de la régénération et de la reconstruction tissulaire, y compris les tissus cutanés et le ligament parodontal; de la prévention de la formation d'adhérences ou encore également dans les pansements pour régénérer les tissus et pour minimiser les risques d'infections.

Dans le domaine dentaire, il est connu d'implanter ce type de membrane entre la gencive et la racine de la dent, en vue de régénérer le ligament parodontal, qui peut être détérioré, par exemple dans les cas de maladies parodontales. La régénération est aussi bien tissulaire qu'osseuse. La membrane permet de guider la régénération.

Dans le domaine maxillofacial, on peut utiliser des membranes pour soulever les sinus.

Ce type de membrane est également utilisé dans diverses spécialités chirurgicales afin d'éviter les adhérences tissulaires.

Dans le domaine de la chirurgie orthopédique par exemple, différentes solutions chirurgicales ont été proposées afin de prévenir la formation d'adhérences après chirurgie : traitement médicamenteux, lambeaux de glissement (graisseux), utilisation de membranes. Les lambeaux locaux peuvent être bénéfiques, mais souvent le résultat est imprédictible. Les membranes sont interposées entre les tissus lésés et les tissus environnants de façon à prévenir les adhérences fibrinotiques, inéluctables après toute chirurgie viscérale, tendineuse et/ou nerveuse. En chirurgie orthopédique, notamment post-traumatique, l'absence de produits efficaces oblige souvent à réaliser des interventions itératives de ténolyse et/ou de neurolyse.

Des membranes collagéniques, conçues pour guider le processus de cicatrisation et prévenir la formation d'adhérences, peuvent être composées à 100 % de collagène. Du fait de son origine animale (bovin ou porcin en général), on ne peut exclure tout risque de contamination lié à l'Encéphalopathie Spongiforme Bovine (ESB) ou maladie de la vache folle.

Il existe encore d'autres membranes synthétiques, et entre autres :
- membrane résorbable composée d'acide hyaluronique et de carboxymethylcellulose,
- membrane de cellulose résorbable,
- barrière non résorbable en poly(tetrafluoroethylène) (PTFE),
- membrane résorbable en poly(éthylène glycol) (PEG).

Il existe donc un grand nombre de membranes différentes, d'origine animale, végétale ou de synthèse, résorbable ou non, sous forme de film ou de tissu.

Concernant les tissus, c'est-à-dire les textiles tissés avec une trame et une chaine, ou encore tricotés, leur fabrication nécessite un ensimage des fils ou fibres, qui consiste à imprégner les fils ou fibres, généralement au moment de l'extrusion, d'une molécule protectrice dite d'ensimage. Cette molécule d'ensimage, généralement une huile de nature organique, confère aux fils ou fibres une certaine tenue et les protègent pendant les diverses manipulations ultérieures (tissage, tricotage, enroulage). Cependant, dans le cas des membranes tissées à implanter dans le corps, il est nécessaire de retirer la molécule d'ensimage pour éviter son « relargage » in situ responsable d'inflammations aigües voire chroniques : on parle alors de désensimage. Cette opération, qui en soi est coûteuse, est délicate industriellement. De plus, un désensimage efficace nécessite l'utilisation de solvants organiques qui peuvent détériorer les fibres de la membrane.

D'autre part, les fibres de la membrane ne confèrent pas en soi des qualités d'anti-adhérence exceptionnelles.

Le brevet WO 2015/075397 A1 divulgue une prothèse de ligament artificiel comprenant un textile de fibres biodégradables. Les fibres sont non tissées, elles sont composées de PCL, de copolymères de PCL et d'acide lactique (L et D) ou d'acide glycolique, des copolymères des acides lactiques et glycoliques (L et D) , de polydioxanone, de polyhydroxyalcanoate et des copolymères de ces différentes molécules. Les fibres comprennent du styrène sulfonate de sodium. Ce dernier est greffé sur les fibres à la suite d'une étape d'ozonation.

Le but de la présente invention est de remédier aux inconvénients de l'art antérieur en définissant une nouvelle membrane qui résout les problèmes liés au désensimage et qui présente de très hautes performances en termes d'anti-adhérence.

Pour ce faire, la présente invention propose une membrane, utilisée notamment en vue de la régénération du ligament parodontal ou de la prévention de la formation d'adhérences, comprenant au moins une nappe de textile formée de fibres réalisées à partir d'au moins un polymère, caractérisé en ce que :
- les fibres sont dépourvues d'ensimage,
- la nappe de textile présente une organisation non tissée de fibres disposées de façon aléatoire ou orientée, et
- les fibres sont revêtues de PolyNaSS (poly (styrène sulfonate de sodium)).

Ainsi, la membrane de l'invention cumule trois caractéristiques distinctes, à savoir l'absence de produit d'ensimage, la nature intissée de la nappe textile et un revêtement, lui conférant les propriétés et avantages suivants :
- Pureté des fibres,
- Isotropie dans le cas de fibres disposées de façon aléatoire,
- Epaisseur variable,
- Homogénéité dans l'épaisseur,
- Anti-adhérence bactérienne du PolyNaSS,
- Régénération et reconstruction tissulaire du PolyNaSS.

Selon une autre caractéristique intéressante de l'invention, le polymère est un polyester, comme par exemple de la poly(ε-caprolactone) (PCL) ou du poly(dioxanone) (PDO). Un mélange de polymères pour former un copolymère est également possible. Un copolymère de poly(ε-caprolactone) (PCL) et de poly(dioxanone) (PDO) est particulièrement avantageux.

En effet, la PCL présente des propriétés non-inflammatoires (car elle ne relargue pas d'acide en se dégradant), c'est un polymère viscoélastique, à biodégradation lente, ce qui est favorable à une reconstruction tissulaire. Le PDO présente aussi des propriétés non-inflammatoires, une biodégradation rapide, et il est facilement éliminé de l'organisme par les urines. Le copolymère PCL-PDO permet de moduler les paramètres des deux polymères pris indépendamment, notamment pour moduler la vitesse de biodégradation.

Le polymère peut également être un polyester, comme par exemple du poly (téréphtalate d'éthylène glycol) (PET) ou encore une polyoléfine, comme par exemple du poly(propylène) (PP). Le PET présente une bonne tenue mécanique. Il est stable en température et aux UV. Le greffage de polyNaSS sur ce matériau est déjà bien maitrisé. Le PP est économique, très résistant à la fatigue et à la flexion, et facilement stérilisable.

Un biopolymère, comme par exemple la cellulose, pourrait aussi être utilisé.

Selon un autre aspect de l'invention, le PolyNaSS est greffé sur les fibres. En variante, le PolyNaSS peut être appliqué sur les fibres par trempage (dipping), pulvérisation (spray), ou tout autre procédé connu.

Selon un mode de réalisation, la nappe de textile présente une épaisseur et une densité de fibre, la densité de fibre pouvant alors varier à travers l'épaisseur avec un gradient progressif ou par palier. A l'inverse, la densité de fibre peut être constante à travers l'épaisseur dans un autre mode de réalisation. Cette capacité de variation de densité de fibre et d'épaisseur n'est pas possible avec un textile tissé.

Selon une forme de réalisation, la membrane peut comprendre une nappe de textile de l'invention disposée sur un substrat. En variante, la membrane peut comprendre deux nappes de textile de l'invention disposées de part et d'autre d'un substrat. Les deux nappes de textile peuvent être identiques ou différentes, aussi bien en épaisseur, en densité, qu'en nature de polymère.

Avantageusement, le substrat est moins poreux que la nappe de textile. Le substrat constitue ainsi une barrière qui empêche ou gêne les tissus de croitre à travers le substrat, évitant ainsi les adhérences.

Le substrat peut être choisi parmi un film de poly(ε-caprolactone) (PCL), un film de poly(dioxanone) (PDO), un film de poly(téréphtalate d'éthylène glycol) (PET), un film de poly(propylène) (PP) ou encore un voile de textile non tissé, par exemple en poly(caprolactone) (PCL) et/ou poly(dioxanone) (PDO), présentant une densité de fibres supérieure à celle de la nappe de textile. Un film ou un voile (tissé ou intissé) de cellulose peut également être utilisé en tant que substrat.

La présente invention définit aussi une structure multicouche comprenant :
- un substrat céramique, avantageusement du phosphate tricalcique, et de préférence du phosphate tricalcique β (βTCP),
- une membrane telle que définie ci-dessus, avantageusement en poly(ε-caprolactone) (PCL), et
- optionnellement, une interface de cohésion, disposée entre le substrat céramique et la membrane.

Cette structure multicouche peut être utilisée pour le traitement de l'arthrose, mais également de la reconstruction cartilagineuse. La présente invention définit aussi un pansement destiné à être appliqué sur une plaie cutanée, le pansement comprenant un substrat et au moins une membrane telle que définie ci-dessus.

La présente invention définit également un procédé de fabrication d'une membrane, utilisée notamment en vue de la régénération du ligament parodontal, comprenant au moins une nappe de textile formée de fibres réalisées à partir d'au moins un polymère, le procédé comprenant les étapes suivantes :
a) se procurer des fibres de polymère dépourvues d'ensimage,
b) déposer ces fibres de polymère de façon aléatoire ou orientée, de manière à former une nappe de textile non tissée,
c) revêtir les fibres de la nappe de textile non tissée, par exemple par greffage ou trempage, de PolyNaSS (polystyrène sulfonate de sodium).

Le polymère peut être de la poly(ε-caprolactone) (PCL), du poly(dioxanone) (PDO), un copolymère de poly(ε-caprolactone) (PCL) et de poly(dioxanone) (PDO), du poly(téréphtalate d'éthylène glycol) (PET) ou du polypropylène (PP).

Une nappe de textile non tissée peut être déposée sur un ou les deux côtés d'un substrat choisi parmi un film de poly(ε-caprolactone) (PCL), un film de poly(dioxanone) (PDO), un film de poly(téréphtalate d'éthylène glycol) (PET), un film de polypropylène (PP) ou encore un voile de textile non tissé, par exemple en poly(ε-caprolactone) (PCL) et/ou poly(dioxanone) (PDO), présentant une densité de fibres supérieure à celle de la nappe de textile.

L'esprit de l'invention réside dans le fait d'allier une nappe intissée dépourvue d'ensimage avec un revêtement anti-adhérence bactérienne de PolyNaSS. La nappe intissée peut être réalisée en polymère ou copolymère biodégradable. Elle peut être associée à un substrat et/ou à une autre nappe intissée. La membrane de l'invention peut encore intégrer d'autres éléments ou composants, comme un substrat céramique. Grâce à l'invention, on peut fabriquer des membranes parodontales, des membranes anti-adhérence, des treillis de renfort de hernies, des pansements ou encore des structures multicouches utilisées pour le traitement des arthroses.

L'invention sera maintenant plus amplement décrite en référence aux dessins joints, donnant à titre d'exemples non limitatifs, plusieurs modes de réalisation de l'invention.

Sur les figures :
La figure 1 est une vue en perspective schématique d'une nappe textile intissée à fibres aléatoires ayant une densité de fibre constante,
La figure 2 est une vue en perspective schématique d'une nappe textile intissée à fibres orientées ayant une densité de fibre constante,
La figure 3 est une vue en coupe fortement agrandie d'une fibre revêtue de PolyNaSS,
La figure 4 est une vue en coupe transversale à travers une nappe textile intissée à fibres aléatoires ayant une densité de fibre croissante ou a gradient progressif,
La figure 5 est une vue en coupe transversale à travers une membrane comprenant un substrat sous forme de film et une nappe textile intissée à fibres aléatoires,
La figure 6 est une vue en coupe transversale à travers une membrane comprenant un substrat sous forme de voile textile intissé à fibres aléatoires et une nappe textile intissée à fibres aléatoires,
La figure 7 est une vue en coupe transversale à travers une membrane comprenant un substrat sous forme de voile textile intissé à fibres aléatoires de densité de fibre constante et deux nappes textile intissées identiques par symétrie à fibres aléatoires de densité de fibre croissante, et
La figure 8 est une vue en coupe transversale à travers une membrane comprenant un substrat sous forme de film et deux nappes textile intissées à fibres aléatoires de densités de fibre constantes différentes.
La figure 9 est une vue en coupe transversale à travers une membrane comprenant un substrat épais sous forme de film et deux nappes textile intissées, dont une à fibres aléatoires et une autre à fibres orientées,
La figure 10 est une vue en perspective schématique d'une nappe textile intissée à fibres aléatoires pourvue de trous,
La figure 11 est une vue en coupe transversale à travers une structure multicouche intégrant la membrane de l'invention,
La figure 12 est une vue similaire à celle de la figure 11 pour une variante de structure multicouche,
La figure 13 est une vue en coupe transversale d'un bloc de βTCP revêtu de PolyNaSS,
La figure 14 est une vue en coupe schématique d'une articulation traitée avec une membrane de l'invention,
La figure 15 est une vue en coupe schématique d'une articulation traitée avec la structure multicouche de la figure 12,
La figure 16 est une vue en coupe schématique d'un os traité avec le bloc de TCP de la figure 13, et
La figure 17 est une vue en coupe transversale d'un pansement intégrant plusieurs membranes de l'invention.

L'entité de base de la membrane de l'invention est une nappe textile non tissée ou intissée réalisée à partir de fibres de polymère disposées de manière aléatoire ou orientée. Par « non tissée ou intissée », on entend tout produit manufacturé à partir de fibres dont la cohésion interne est assurée par des méthodes mécaniques et/ou physiques, et/ou chimiques et/ou par combinaison de ces divers procédés, à l'exclusion du tissage et du tricotage. Les fibres peuvent être orientées dans une direction particulière ou au hasard sous la forme d'un enchevêtrement non répétitif. Le feutre peut être considéré comme un non-tissé ou intissé.

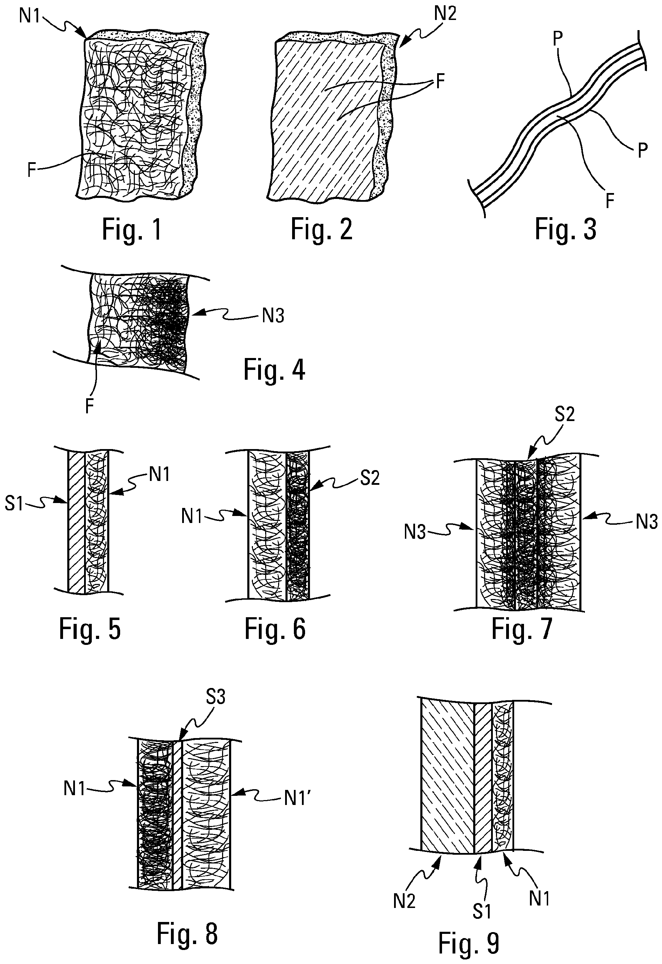

Sur la figure 1, on voit un morceau de nappe textile non tissée ou intissée dont les fibres F sont disposées de manière aléatoire, alors que sur la figure 2, les fibres F sont disposées de manière orientée. Toutes les fibres F peuvent avoir la même orientation, ou en variante, elles peuvent avoir des orientations déterminées différentes, par exemple d'une couche à l'autre.

Selon l'invention, les fibres F sont dépourvues d'ensimage et sont donc pures. En effet, il n'est pas nécessaire de les ensimer, comme ce serait le cas avec une nappe textile tissée, étant donné qu'elles ne sont pas soumises à des contraintes importantes : elles sont simplement déposées les unes sur les autres, et éventuellement comprimées.

Les fibres F sont réalisées à partir de polymère(s) ou de copolymère(s), ou même de biopolymère. On peut par exemple utiliser un polyester, comme la poly(caprolactame) (PCL) ou le poly(dioxanone) (PDO). Un copolymère de poly(ε-caprolactone) (PCL) et de poly(dioxanone) (PDO) est un bon candidat, car le PCL et le PDO sont tous deux biodégradables, mais avec des temps de dégradation différents, le PCL se dégradant plus vite que le PDO. Leur copolymère présente donc une durée de dégradation plus longue et échelonnée.

La poly(caprolactone) (PCL) est un polyester biodégradable comportant une faible température de fusion d'environ 60 °C et une température de transition vitreuse d'environ -60 °C. On peut aussi la désigner sous le terme de poly(ε-caprolactone).

D'autres fibres polyesters, biodégradables ou non, peuvent également être utilisées, comme par exemple le poly (téréphtalate d'éthylène glycol) (PET).

On peut aussi utiliser une polyoléfine, comme le poly(propylène) (PP).

La cellulose peut aussi être utilisée.

Selon l'invention, les fibres sont revêtues de PolyNaSS (poly (styrène sulfonate de sodium)). Le PolyNaSS peut par exemple être greffé sur les fibres. N'importe quel procédé ou technique de greffage peut être utilisé, comme par exemple celui décrit dans le document FR3042715, qui décrit un procédé de greffage de PolyNaSS sur des implants en titane, mais ce procédé peut également être mis en oeuvre sur des membranes de l'invention. Le procédé de greffage de ce document peut être adapté au greffage de fibres et peut dans ce cas prévoir les étapes successives suivantes :
a) Monter les membranes sur une structure de support,
b) Activer la surface des fibres en générant des fonctions peroxydes ou hydroperoxydes par ozonation, ou par irradiation UV,
c) Introduire les membranes (encore montées sur leur support, ou un autre support, ou sur aucun support) dans une enceinte de polymérisation étanche remplie de gaz inerte, tel que l'argon : un autre gaz inerte peut aussi être utilisé,
d) Monter les membranes (montées sur leur support, ou un autre support, ou sur aucun support) sur un ascenseur installé dans l'enceinte étanche,
e) Actionner l'ascenseur pour plonger les membranes dans un bain de polymérisation, par exemple de monomère, comme le styrène sulfonate de sodium (NaSS), présent dans l'enceinte,
f) Soumettre le bain de polymérisation à une énergie, par exemple thermique ou UV, pour polymériser le monomère bioactif à partir de la surface des fibres, et ainsi obtenir une membrane revêtue d'une couche de polymère greffé, par exemple du PolyNaSS,
g) Remonter l'ascenseur pour extraire les membranes revêtues du bain de polymérisation,
h) Retirer les membranes de l'ascenseur,
i) Extraire les membranes de l'enceinte étanche,
j) Laver éventuellement les membranes, par exemple par aspersion d'eau pure, pour les débarrasser de l'excès de polymère bioactif non greffé,
k) Sécher éventuellement les membranes greffées.

Cette succession d'étapes permet une mise en oeuvre industrialisée du procédé de greffage. La plupart des étapes sont essentielles, voire indispensables pour une mise en oeuvre industrialisée, reproductible, efficace, rapide et fiable du procédé de greffage de polymère bioactif.

A la place du greffage, on peut aussi appliquer le PolyNaSS sur les fibres de la membrane en utilisant par exemple des techniques de trempage (dipping) ou de pulvérisation (spray).

Le PolyNaSS est connu pour ses qualités d'anti-adhésion bactérienne, d'où un risque moins élevé d'adhésion de bactéries pouvant causer des infections et pour ses qualités de régénération, de réparation et de reconstruction tissulaire.

Ainsi, l'invention propose une membrane synthétique intégrant une nappe textile intissée de fibres non ensimées revêtues de PolyNaSS. Sur la figure 3, on voit une section de fibre F revêtue d'une couche P de PolyNaSS : il n'y a pas de couche d'ensimage.

Sur la figure 4, on voit une nappe textile intissée de fibres non ensimées revêtues de PolyNaSS disposées de manière aléatoire. Toutefois, on peut remarquer que la densité des fibres n'est pas constante sur l'épaisseur de la nappe : au contraire, elle varie selon un gradient continu progressif de la gauche vers la droite sur la figure 4. La progression du gradient peut être linéaire ou au contraire par palier. On pourrait envisager une nappe avec plusieurs couches de densités différentes.

La membrane de l'invention, selon un mode de réalisation simple, peut uniquement être constituée par une nappe textile intissée de fibres non ensimées revêtues de PolyNaSS, comme celles illustrées sur les figures 1, 2 et 4. L'épaisseur de la nappe textile peut être de 0,1 à 3 mm, avantageusement de 0,1 à 0,8 mm, et de préférence de 0,2 à 0,4 mm.

La nappe peut cependant être alliée à d'autre éléments ou composants. Les figures 5 à 9 illustrent des membranes complexes selon l'invention. Une nappe selon l'invention peut être associée à un substrat et/ou à une autre nappe de l'invention.

Le substrat peut être de toute nature, structure, épaisseur et forme. Il peut par exemple être choisi parmi un film de poly(ε-caprolactone) (PCL), un film de poly(dioxanone) (PDO), un film de poly(téréphtalate d'éthylène glycol) (PET), un film de poly(propylène) (PP) ou encore un voile de textile non tissé, par exemple en poly(ε-caprolactone) (PCL) et/ou poly(dioxanone) (PDO), présentant une densité de fibres supérieure à celle de la nappe de textile. Un film ou un voile (tissé ou intissé) de cellulose peut également être utilisé en tant que substrat. Avantageusement, le substrat est moins poreux que la nappe. Ainsi, il peut jouer un rôle de barrière. L'épaisseur du film peut être de 0,05 à 0,5 mm, avantageusement de 0,05 à 0,3 mm, et de préférence de 0,05 à 0,25 mm.

Sur la figure 5, la membrane comprend un substrat S1 sous la forme de film et une nappe textile intissée N1 à fibres aléatoires de densité constante, qui peut être identique ou similaire à celle de la figure 1. La nappe est plus épaisse que le substrat. La nappe N1 peut être simplement déposée sur le film S1 ou en variante, la nappe N1 peut être collée sur le substrat S1, ou inversement.

Sur la figure 6, la membrane comprend un substrat S2 sous la forme d'un voile textile intissé à fibres aléatoires et une nappe textile intissée N1 à fibres aléatoires de densité constante, qui peut être identique ou similaire à celle de la figure 1. Le substrat S2 et la nappe N1 ont donc une structure intissée identique. Cependant, on peut remarquer sur la figure 6 que la densité de fibre du substrat S2 est supérieure à celle de la nappe N1. On peut aussi dire que la nappe N1 est plus poreuse que le substrat S2.

Sur la figure 7, la membrane comprend un substrat S2 sous la forme de voile textile intissé à fibres aléatoires de densité de fibre constante et deux nappes textiles intissées N3 identiques par symétrie à fibres aléatoires, mais de densité de fibre croissante. Les nappes N3 peuvent être identiques ou similaires à celle de la figure 4. La densité de fibre du substrat S2 est supérieure ou égale à la densité maximale des nappes N3.

Sur la figure 8, la membrane comprend un substrat S3 sous la forme d'un film mince et deux nappes textile intissées N1 et N1' à fibres aléatoires de densités de fibre constantes différentes. En effet, on peut voir que la densité de la nappe N1 est supérieure à celle de la nappe N1'.

Sur la figure 9, la membrane comprend un substrat sous la forme d'un film épais et deux nappes textile intissées N1 et N2, dont une à fibres aléatoires et l'autre à fibres orientées. La nappe N1 peut être identique ou similaire à celle de la figure 1 et la nappe N1 peut être identique ou similaire à celle de la figure 2.

Dans les exemples des figures 5 à 9, l'épaisseur totale de la membrane multicouche pout varier de 0,1 à 3 mm, avantageusement de 0,1 à 0,8 mm, et de préférence de 0,2 à 0,4 mm.

A partir de ces multiples exemples, on comprend qu'il est possible de réaliser une multitude de membranes de conceptions différentes, allant d'une simple nappe à un assemblage de nappe(s) et de substrat(s). On peut prévoir plus de deux nappes et plus d'un substrat, sans sortir du cadre de l'invention.

La nappe textile de l'invention peut être réalisée à partir des fibres (F) de polymère dépourvues d'ensimage, qui sont déposées sans tissage ni tricotage de façon aléatoire ou orientée, afin d'obtenir une nappe textile non tissée ou intissée ayant une épaisseur et une densité constantes ou progressives. Ensuite, du PolyNaSS (poly (styrène sulfonate de sodium)) est appliqué (greffage, trempage, pulvérisation) sur les fibres de la nappe de textile non tissée. La nappe peut être formée directement sur le substrat de manière à y adhérer mécaniquement et/ou chimiquement.

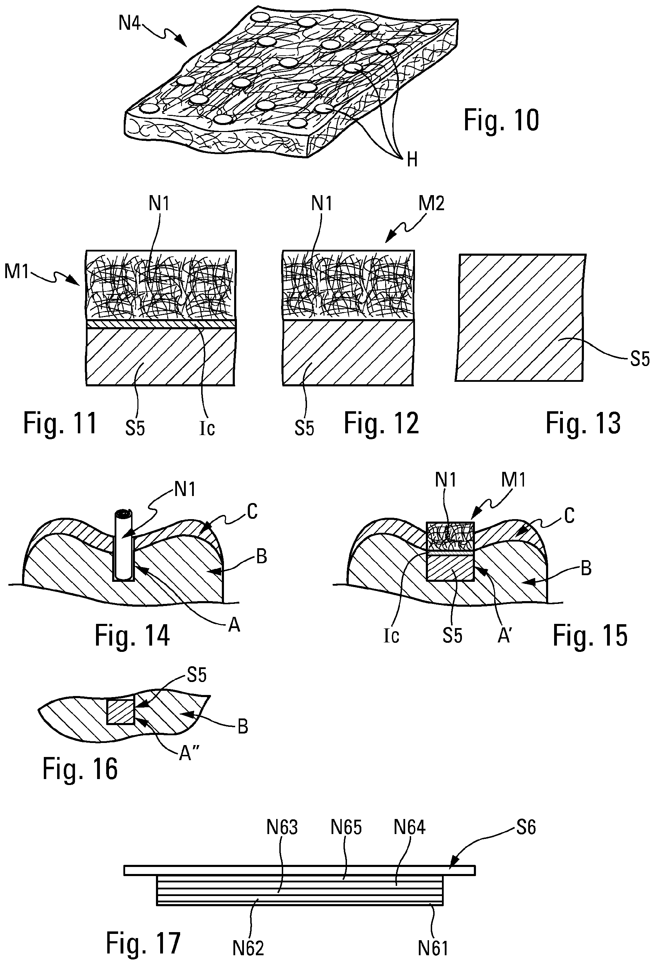

Sur la figure 10, on voit une membrane N4, formée d'une nappe de textile non tissée revêtue de PolyNaSS, qui comprend des trous H, avantageusement disposés de manière régulière sous la forme d'un réseau. La membrane est ainsi ajourée. Les trous H peuvent présenter une section ronde, carrée, rectangulaire, hexagonale, etc. Les trous peuvent représenter jusqu'à 90 % de la surface de la membrane, de sorte qu'elle ressemble alors à un treillis ou une résille. Les trous H peuvent être réalisés par poinçonnage ou découpe laser par exemple. La membrane peut être plane ou calandrée. Une telle membrane peut être utilisée comme treillis de renfort de hernie. Le risque d'infection est diminué de par la présence du PolyNaSS et la reconstruction tissulaire se fait à travers les trous H.

La figure 11 montre de manière très schématique une structure multicouche complexe M1 comprenant :
- un substrat céramique S5, qui peut comprendre du ou être constitué de phosphate tricalcique, et plus particulièrement de phosphate tricalcique β (βTCP),
- une interface de cohésion Ic, qui peut être réalisée par imprégnation d'un polymère, tel que de la poly(ε-caprolactone) (PCL), et
- une membrane, formée d'une nappe de textile non tissée revêtue de PolyNaSS, telle que la membrane N1 de la figure 1.

Les phosphates tricalciques sont des solides blanchâtres : ils composent la partie minérale de l'os et des dents. Le phosphate tricalcique β (βTCP) est une céramique macroporeuse dont la taille des pores peut varier de 250 à 400 µm. La stérilisation est assurée par irradiation gamma. Cette céramique est parfaitement biocompatible, bioactive et d'origine synthétique, ce qui évite tout problème d'intolérance immunitaire et élimine tout risque de transmission virale. Par ailleurs, elle est ostéo-conductrice et résorbable.

L'interface de cohésion Ic peut être réalisée par application d'un polymère liquide sur une face du substrat S5. Les polymères susmentionnés peuvent être utilisés, avec une préférence pour la poly(ε-caprolactone) (PCL). La fonction de cette interface de cohésion Ic est d'assurer la liaison mécanique entre le substrat S5 et la membrane de l'invention.

La membrane de l'invention peut être l'une quelconque des figures 1, 2, 3, 6 ou 7, ou encore une autre membrane monocouche ou multicouche non tissée revêtue de PolyNaSS. Seule la membrane de l'invention peut être revêtue de PolyNaSS. En variante, toute la structure multicouche complexe peut être revêtue de PolyNaSS.

La figure 12 montre de manière très schématique une variante de structure multicouche M2, moins complexe que celle de la figure 11, puisqu'elle ne comprend pas d'interface de cohésion. La membrane de l'invention est alors directement en contact du substrat S5. La membrane peut par exemple être directement formée sur le substrat S5 par dépôt successif de fibres. Le revêtement de PolyNaSS, pur ou additivé, peut ensuite être greffé, trempé ou pulvérisé, sur la membrane et le substrat : il peut éventuellement contribuer à la liaison entre la membrane et le substrat S5.

La figure 13 montre de manière très schématique le substrat S5 revêtu de PolyNaSS. De préférence, le substrat céramique S5 est un substitut osseux et peut comprendre du ou être constitué de phosphate tricalcique, et plus particulièrement de phosphate tricalcique β (βTCP). Le PolyNaSS est de préférence greffé, mais il peut aussi être appliqué par trempage ou pulvérisation dans certains cas. Une protection pourrait être recherchée pour un tel substrat céramique revêtu de PolyNaSS.

Les figures 14 et 15 illustrent des procédés de traitement mettant en oeuvre la membrane de l'invention sous des formes différentes. On y voit une partie osseuse comprenant de l'os spongieux B et du cartilage C qui recouvre l'os spongieux B. En cas de détérioration du cartilage C, et éventuellement de l'os spongieux B, par exemple due à l'arthrose ou à un traumatisme, il est prévu selon l'invention de réaliser un alésage A, A' qui traverse la cartilage C et éventuellement l'os spongieux B, comme cela est représenté sur les figures 14 et 15. Sans sortir du cadre de l'invention, l'alésage A, A' pourrait s'arrêter avant d'atteindre l'os spongieux B, de sorte que le fond de l'alésage serait formé par du cartilage C.

Dans le cas de la figure 14, une membrane de l'invention est insérée dans l'alésage A. La membrane peut être l'une quelconque des figures 1, 2, 3, 6 ou 7, ou encore une autre membrane monocouche ou multicouche non tissée revêtue (greffée, trempée ou pulvérisée) de PolyNaSS. La membrane peut être enroulée sur elle-même pour former un petit cylindre que l'on insère ensuite dans l'alésage A. Après insertion complète, la membrane se détend dans l'alésage en se plaquant contre la paroi interne de l'alésage A. Le petit cylindre de membrane peut faire saillie hors de l'alésage A, comme on peut le voir sur la figure 14, ou, en variante, le petit cylindre peut être ajusté ou coupé pour qu'il vienne sensiblement en affleurement avec la surface externe du cartilage C. A la place du cylindre, on peut aussi envisager de former une membrane qui s'adapte à l'alésage A : la membrane peut présenter la forme d'une petite tige. Plus simplement, on peut enfoncer (par bourrage) une membrane dans l'alésage A et couper ou non ce qui dépasse.

Ici, l'intérêt est la prévention de l'infection pour l'ensemble du dispositif, la reconstruction cartilagineuse et in fine la diminution de l'arthrose.

Dans le cas de la figure 15, une petite tige formée à partir de la structure multicouche complexe M1 est insérée dans l'alésage A'. Le substrat S5 est disposé dans l'os spongieux B, alors que la membrane N1 est disposée dans le cartilage C. L'interface de cohésion Ic se situe environ au niveau de l'interface entre l'os spongieux B et le cartilage C.

Ici, l'intérêt est la prévention de l'infection pour l'ensemble du dispositif, la reconstruction cartilagineuse, à laquelle s'ajoute l'amélioration de la reconstruction osseuse pour la partie os et in fine la diminution de l'arthrose.

La figure 16 montre un os B dans lequel un alésage A" a été percé, puis rempli avec une pastille ou une tige de substrat S5 de la figure 13, à savoir une céramique revêtue de PolyNaSS, et de préférence du phosphate tricalcique β (βTCP) greffé, trempé ou pulvérisé de PolyNaSS.

Cette technique remplace avantageusement les différentes techniques de réparation par des chondrocytes autologues obtenus par cultures (technique Carticel ^{®}). Toutes ces techniques y compris celle décrite dans le brevet visent à obtenir un cartilage sain et non un fibrocartilage.

Ici, l'intérêt est clairement la prévention de l'infection à laquelle s'ajoute l'amélioration de la reconstruction osseuse.

La membrane de l'invention peut également servir à la conception de pansements de plaies cutanées, destinés à protéger des plaies diverses, et plus particulièrement les plaies étendues, telles que les brulures, les écorchures, les ulcères variqueux, etc. Un pansement selon l'invention comprend un substrat S6 et une, mais de préférence plusieurs, membranes de l'invention N61 à N65. Le substrat S6 peut être tout à fait classique et se présenter sous la forme d'une bande souple de matière plastique. Les membranes peuvent être identiques, mais de préférence, elles sont différentes. Par exemple, la membrane la plus externe 61 peut être très fine et plus fortement greffée (taux de greffage élevé) que la seconde membrane 62. La porosité ou densité de la membrane 61 peut également être supérieure à celle de la seconde membrane 62. Les membranes 62 à 65 peuvent être identiques ou différentes à la manière de la membrane 61 par rapport à la membrane 62. On obtient ainsi une sorte de millefeuille de membranes de caractéristiques distinctes. Les membranes peuvent être réalisées à partir de nappe intissée de polymère, tel que défini précédemment. De préférence, le polymère est de la poly(ε-caprolactone) (PCL).

En pratique, après avoir gardé ce pansement sur une plaie pendant un certain temps, il est possible de le retirer en laissant une ou plusieurs membranes en place, qui vont ainsi constituer une sorte de seconde peau. On peut dire que les membranes sont « pelables », avec la membrane externe 61 qui forme une fine peau artificielle à haute teneur en PolyNaSS. Cela permet de réduire considérablement les risques d'infection, tout en accélérant la régénération tissulaire.

Il faut également noter que le taux de greffage de polyNaSS est variable d'une application à l'autre. Par exemple, on peut prévoir des taux de greffage très faibles, proches de zéro, pour l'antiadhérence cellulaire, parce que l'ozonisation permet de créer des hydroperoxydes qui seront hydrolysés et généreront des OH en surface qui les rendront hydrophiles et donc non adhérents pour les cellules. En pratique, un taux de greffage de polyNaSS de 0,01 à 0,1 micromoles par g ou cm2 donne de bons résultats pour l'antiadhérence cellulaire. A l'inverse, on peut prévoir des taux de greffage très élevés, jusqu'à 40 micromoles par g ou cm2 pour l'anti-adhérence bactérienne et la régénération tissulaire. En pratique, un taux de greffage de polyNaSS de 12 micromoles par g ou cm2 donne de bons résultats pour l'anti-adhérence bactérienne.

Ainsi, grâce à l'invention, on dispose d'une membrane synthétique qui peut être utilisée en chirurgie dentaire en vue de la régénération du ligament parodontal, mais également dans diverses spécialités chirurgicales pour empêcher la formation d'adhérences ou encore en vue de la régénération et la reconstruction tissulaire (treillis de renfort pour les hernies), y compris les tissus cutanés, par exemple sous la forme de pansements pour minimiser les risques d'infections et accélérer la régénération.

## Revendications

1. Membrane, utilisée notamment en vue de la régénération du ligament parodontal, de la prévention de la formation d'adhérences ou de la régénération et la reconstruction tissulaire, comprenant au moins une nappe de textile (N1, N1', N2, N3 ; N4 ; N61, N62, N63, N64, N65) formée de fibres (F) réalisées à partir d'au moins un polymère, **caractérisée en ce que** :
- les fibres (F) sont dépourvues d'ensimage,
- la nappe de textile (N1, N1', N2, N3 ; N4 ; N61, N62, N63, N64, N65) présente une organisation non tissée de fibres disposées de façon aléatoire ou orientée, et
- les fibres (F) sont revêtues de PolyNaSS (polystyrène sulfonate de sodium).

2. Membrane selon la revendication 1, dans laquelle le polymère est choisi parmi la poly(ε-caprolactone) (PCL), le poly(dioxanone) (PDO), un copolymère de poly(ε-caprolactone) (PCL) et de poly(dioxanone) (PDO), le poly(téréphtalate d'éthylène glycol) (PET), et le polypropylène (PP).

3. Membrane selon l'une quelconque des revendications précédentes, dans laquelle la nappe de textile (N4) comprenant des trous (H).

4. Membrane selon l'une quelconque des revendications précédentes, dans laquelle la nappe de textile (N3) présente une épaisseur et une densité de fibre, la densité de fibre variant à travers l'épaisseur avec un gradient progressif.

5. Membrane selon l'une quelconque des revendications précédentes, dans laquelle la nappe de textile (N1, N1', N2, N3 ; N61, N62, N63, N64, N65) est disposée sur un substrat (S1, S2, S3).

6. Membrane selon l'une quelconque des revendications précédentes, comprenant deux nappes de textile (N1, N1', N2, N3) disposées de part et d'autre d'un substrat (S1, S2, S3).

7. Membrane selon la revendication 5 ou 6, dans laquelle le substrat (S1, S2, S3) est moins poreux que la nappe de textile (N1, N1', N2, N3).

8. Membrane selon la revendication 5, 6 ou 7, dans laquelle le substrat (S1, S2, S3) est choisi parmi :
- un film de poly(ε-caprolactone) (PCL),
- un film de poly(dioxanone) (PDO),
- un film de polytéréphtalate d'éthylène (PET),
- un film de polypropylène (PP),
- un voile de textile non tissé, avantageusement en poly(ε-caprolactone) (PCL) et/ou poly(dioxanone) (PDO), présentant une densité de fibres supérieure à celle de la nappe de textile (N1, N1', N2, N3).

9. Structure multicouche (M1 ; M2) comprenant :
- un substrat céramique (S5), avantageusement du phosphate tricalcique, et de préférence du phosphate tricalcique β (βTCP),
- une membrane selon l'une quelconque des revendications précédentes, avantageusement en poly(ε-caprolactone) (PCL), et
- optionnellement, une interface de cohésion (Ic), disposée entre le substrat céramique (S5) et la membrane.

10. Pansement destiné à être appliqué sur une plaie cutanée, le pansement comprenant un substrat (S6) et au moins une membrane selon l'une quelconque des revendications 1 à 8.

11. Procédé de fabrication d'une membrane, utilisée notamment en vue de la régénération du ligament parodontal, comprenant au moins une nappe de textile (N1, N1', N2, N3; N4 ; N61, N62, N63, N64, N65) formée de fibres (F) réalisées à partir d'au moins un polymère, le procédé comprenant les étapes suivantes :
a) Se procurer des fibres (F) de polymère dépourvues d'ensimage,
b) Déposer ces fibres (F) de polymère de façon aléatoire ou orientée, de manière à former une nappe de textile non tissée (N1, N1', N2, N3; N4 ; N61, N62, N63, N64, N65),
c) Appliquer du PolyNaSS (polystyrène sulfonate de sodium) sur les fibres de la nappe de textile non tissée (N1, N1', N2, N3 ; N4 ; N61, N62, N63, N64, N65).

12. Procédé selon la revendication 11, dans lequel le polymère est choisi parmi la poly(ε-caprolactone) (PCL), le poly(dioxanone) (PDO), un copolymère de poly(ε-caprolactone) (PCL) et de poly(dioxanone) (PDO), le poly(téréphtalate d'éthylène glycol) (PET), et le polypropylène (PP).

13. Procédé selon la revendication 11 ou 12, dans lequel une nappe de textile non tissée (N1, N1', N2, N3) est déposée sur un ou les deux côtés d'un substrat (S1, S2, S3) choisi parmi :
- un film de poly(ε-caprolactone) (PCL),
- un film de poly(dioxanone) (PDO),
- un film de polytéréphtalate d'éthylène (PET),
- un film de polypropylène (PP),
- un voile de textile non tissé, avantageusement en poly(ε-caprolactone) (PCL) et/ou poly(dioxanone) (PDO), présentant une densité de fibres supérieure à celle de la nappe de textile (N1, N1', N2, N3).

## Patentansprüche

1. Membran, insbesondere zur Verwendung für die Regeneration des parodontalen Ligaments, zur Verhinderung der Bildung von Adhäsionen oder zur Regeneration und zum Wiederaufbau von Gewebe, umfassend mindestens ein Textilvlies (N1, N1', N2, N3; N4; N61, N62, N63, N64, N65), das aus Fasern (F) gebildet ist, die aus mindestens einem Polymer hergestellt sind, **dadurch gekennzeichnet, dass**
- die Fasern (F) frei von Schlichte sind,
- das Textilvlies (N1, N1', N2, N3; N4; N61, N62, N63, N64, N65) eine nicht gewebte Struktur von Fasern aufweist, die zufällig oder orientiert angeordnet sind, und
- die Fasern (F) mit PolyNaSS (Natriumpolystyrolsulfonat) beschichtet sind.

2. Membran nach Anspruch 1, bei dem das Polymer ausgewählt ist aus Poly(ε-caprolacton) (PCL), Poly(dioxanon) (PDO), einem Copolymer von Poly(ε-caprolacton) (PCL) und Poly(dioxanon) (PDO), Poly(ethylenglycolterephthalat) (PET), und Polypropylen (PP).

3. Membran nach einem der vorhergehenden Ansprüche, bei der das Textilvlies (N4) Löcher (H) umfasst.

4. Membran nach einem der vorhergehenden Ansprüche, bei der das Textilvlies (N3) eine Dicke und eine Faserdichte aufweist, wobei sich die Faserdichte über die Dicke hinweg mit progressivem Gradienten ändert.

5. Membran nach einem der vorhergehenden Ansprüche, bei der das Textilvlies (N1, N1', N2, N3; N61, N62, N63, N64, N65) auf einem Substrat (S1, S2, S3) angeordnet ist.

6. Membran nach einem der vorhergehenden Ansprüche, umfassend zwei Textilvliese (N1, N1', N2, N3) umfasst, die auf beiden Seiten eines Substrats (S1, S2, S3) angeordnet sind.

7. Membran nach Anspruch 5 oder 6, bei der das Substrat (S1, S2, S3) weniger porös ist als das Textilvlies (N1, N1', N2, N3).

8. Membran nach Anspruch 5, 6 oder 7, bei der das Substrat (S1, S2, S3) ausgewählt ist aus:
- einer Poly(ε-caprolacton)-Folie (PCL-Folie),
- einer Poly(dioxanon)-Folie (PDO-Folie),
- einer Polyethylenterephthalat-Folie (PET-Folie),
- einer Polypropylen-Folie (PP-Folie),
- einem nicht gewebten textilen Flächengebildes, vorzugsweise aus Poly(ε-caprolacton) (PCL) und/oder Poly(dioxanon) (PDO), mit einer höheren Faserdichte als derjenigen des Textilvlieses (N1, N1', N2, N3).

9. Mehrschichtige Struktur (M1; M2), umfassend:
- ein Keramiksubstrat (S5), vorteilhafterweise aus Tricalciumphosphat und vorzugsweise ß-Tricalciumphosphat (βTCP),
- eine Membran nach einem der vorhergehenden Ansprüche, vorzugsweise aus Poly(ε-caprolacton) (PCL), und
- wahlweise eine Kohäsionsgrenzfläche (Ic), die zwischen dem Keramiksubstrat (S5) und der Membran angeordnet ist.

10. Verband zum Aufbringen auf eine Hautwunde, wobei der Verband ein Substrat (S6) und mindestens eine Membran nach einem der Ansprüche 1 bis 8 umfasst.

11. Verfahren zur Herstellung einer Membran zur Verwendung insbesondere für die Regeneration des parodontalen Ligaments, mit mindestens einem Textilvlies (N1, N1', N2, N3; N4; N61, N62, N63, N64, N65), das aus Fasern (F) gebildet ist, die aus mindestens einem Polymer hergestellt sind, wobei das Verfahren die folgenden Schritte umfasst:
a) Beschaffen von Polymerfasern (F), die frei von Schlichte sind,
b) Ablegen dieser Polymerfasern (F) auf zufällige oder ausgerichtete Weise zur Bildung eines nicht gewebten Textilvlieses (N1, N1', N2, N3; N4; N61, N62, N63, N64, N65),
c) Aufbringen von PolyNaSS (Natriumpolystyrolsulfonat) auf die Fasern des nicht gewebten Textilvlieses (N1, N1', N2, N3; N4; N61, N62, N63, N64, N65).

12. Verfahren nach Anspruch 11, bei dem das Polymer ausgewählt ist aus Poly(ε-caprolacton) (PCL), Poly(dioxanon) (PDO), einem Copolymer von Poly(ε-caprolacton) (PCL) und Poly(dioxanon) (PDO), Poly(ethylenglykolterephthalat) (PET) und Polypropylen (PP).

13. Verfahren nach Anspruch 11 oder 12, bei dem ein nicht gewebtes Textilvlies (N1, N1', N2, N3) auf einer oder beiden Seiten eines Substrats (S1, S2, S3) abgelegt wird, das ausgewählt ist aus:
- einer Poly(ε-caprolacton)-Folie (PCL-Folie),
- einer Poly(dioxanon)-Folie (PDO-Folie),
- einer Polyethylenterephthalat-Folie (PET-Folie),
- einer Polypropylen-Folie (PP-Folie),
- einem nicht gewebten textilen Flächengebilde, vorzugsweise aus Poly(ε-caprolacton) (PCL) und/oder Poly(dioxanon) (PDO), mit einer höheren Faserdichte als derjenigen des Textilvlieses (N1, N1', N2, N3).

## Claims

1. A membrane, used in particular for regenerating the periodontal ligament, preventing the formation of adhesions or tissue regeneration and reconstruction, comprising at least one layer of fabric (N1, N1', N2, N3; N4; N61, N62, N63, N64, N65) made from fibres (F) produced from at least one polymer, **characterised in that**:
- the fibres (F) are free of sizing,
- the layer of fabric (N1, N1', N2, N3; N4; N61, N62, N63, N64, N65) has a non-woven arrangement of fibres disposed in a random or aligned manner, and
- the fibres (F) are coated with PolyNaSS (polystyrene sodium sulfonate).

2. The membrane according to claim 1, wherein the polymer is selected from poly(ε-caprolactone) (PCL), poly(dioxanone) (PDO), a copolymer of poly(ε-caprolactone) (PCL) and poly (dioxanone) (PDO), polyethylene glycol terephthalate (PET), and polypropylene (PP).

3. The membrane according to any one of the preceding claims, wherein the layer of fabric (N4) comprising holes (H).

4. The membrane according to any one of the preceding claims, wherein the layer of fabric (N3) has a thickness and a fibre density, the fibre density varying through the thickness with a progressive gradient.

5. The membrane according to any one of the preceding claims, wherein the layer of fabric (N1, N1', N2, N3; N61, N62, N63, N64, N65) is disposed on a substrate (S1, S2, S3).

6. The membrane according to any one of the preceding claims, comprising two layers of fabric (N1, N1', N2, N3) disposed on either side of a substrate (S1, S2, S3).

7. The membrane according to claim 5 or 6, wherein the substrate (S1, S2, S3) is less porous than the layer of fabric (N1, N1', N2, N3).

8. The membrane according to claim 5, 6 or 7, wherein the substrate (S1, S2, S3) is chosen from among:
- a poly(ε-caprolactone) (PCL) film,
- a poly(dioxanone) (PDO) film,
- a polyethylene terephthalate (PET) film,
- a polypropylene (PP) film,
- a non-woven fabric web, advantageously made of poly(ε-caprolactone) (PCL) and/or poly(dioxanone) (PDO), having a greater fibre density than that of the layer of fabric (N1, N1', N2, N3).

9. A multilayer structure (M1; M2) comprising:
- a ceramic substrate (S5), advantageously tricalcium phosphate, and preferably tricalcium β-phosphate (βTCP),
- a membrane according to any one of the preceding claims, advantageously made of poly(ε-caprolactone) (PCL), and
- optionally, a cohesion interface (Ic), disposed between the ceramic substrate (S5) and the membrane.

10. A dressing intended to be applied onto a skin wound, the dressing comprising a substrate (S6) and at least one membrane according to any one of claims 1 to 8.

11. A method of manufacturing a membrane, used in particular for regenerating the periodontal ligament, comprising at least one layer of fabric (N1, N1', N2, N3; N4; N61, N62, N63, N64, N65) made of fibres (F) produced from at least one polymer, the method comprising the following steps:
- obtaining polymer fibres (F) free of sizing,
- depositing these polymer fibres (F) in a random or aligned manner, so as to form a non-woven layer of fabric (N1, N1', N2, N3; N4; N61, N62, N63, N64, N65),
- applying PolyNaSS (polystyrene sodium sulfonate) on the fibres of the non-woven layer of fabric (N1, N1', N2, N3; N4; N61, N62, N63, N64, N65).

12. The method according to claim 11, wherein the polymer is selected from poly(ε-caprolactone) (PCL), poly(dioxanone) (PDO), a copolymer of poly(ε-caprolactone) (PCL) and poly (dioxanone) (PDO), polyethylene glycol terephthalate (PET), and polypropylene (PP).

13. The method according to claim 11 or 12, wherein a non-woven layer of fabric (N1, N1', N2, N3) is deposited on one or both sides of a substrate (S1, S2, S3) selected from among:
- a poly(ε-caprolactone) (PCL) film,
- a poly(dioxanone) (PDO) film,
- a polyethylene terephthalate (PET) film,
- a polypropylene (PP) film,
- a non-woven fabric web, advantageously made of poly(ε-caprolactone) (PCL) and/or poly(dioxanone) (PDO), having a greater fibre density than that of the layer of fabric (N1, N1', N2, N3).
